# EUROPEAN PATENT SPECIFICATION

(11) **EP 1 981 405 B1**
(45) Date of publication and mention of the grant of the patent: **04.04.2012**
(21) Application number: 07811782.7
(22) Date of filing: 12.01.2007
(51) Int. Cl.: A61B 8/00

(54) **CONTROL PANEL FOR A MEDICAL IMAGING SYSTEM**
STEUERTAFEL FÜR EIN MEDIZINISCHES BILDGEBUNGSSYSTEM
PANNEAU DE COMMANDE POUR SYSTEME D'IMAGERIE MEDICALE

(30) Priority: 13.01.2006 US 331651
(43) Date of publication of application: 22.10.2008
(73) Proprietor: Boston Scientific Limited, Hastings Christ Church (BB)
(72) Inventor: RAMOS, Michael T., San Jose, California 95119 (US)
(74) Representative: Pfenning, Meinig & Partner GbR
(86) International application number: PCT/US2007/060492
(87) International publication number: WO 2007/133818

(56) References cited:
- WO-A-00/19907
- US-A1- 2004 019 255
- US-A1- 2004 024 288
- US-A1- 2005 228 276

## Description

### FIELD OF THE INVENTION

The field of the invention relates to medical imaging systems, and more particularly to a control panel for a medical imaging system.

### BACKGROUND OF THE INVENTION

Intraluminal, intracavity, intravascular, and intracardiac treatments and diagnosis of medical conditions utilizing minimally invasive procedures are effective tools in many areas of medical practice. These procedures are typically performed using imaging and treatment catheters that are inserted percutaneously into the body and into an accessible vessel of the vascular system at a site remote from the vessel or organ to be diagnosed and/or treated, such as the femoral artery. The catheter is then advanced through the vessels of the vascular system to the region of the body to be treated. The catheter may be equipped with an imaging device, typically an ultrasound imaging device, which is used to locate and diagnose a diseased portion of the body, such as a stenosed region of an artery. For example, U.S. Pat. No. 5,368,035, issued to Hamm et al., describes a catheter having an intravascular ultrasound imaging transducer.

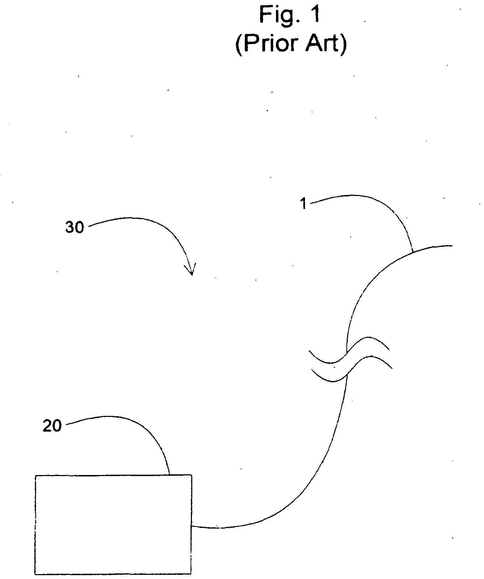

The imaging device is generally part of an imaging system 30, an example of which is shown in Fig. 1. A typical imaging system 30 includes an imaging transducer assembly and coupled to the imaging transducer assembly 1, an imaging console 20 having a display screen, a processor and associated graphics hardware (not shown). To form an image of body tissue by an intravascular ultrasound system (IVUS), the imaging transducer assembly 1 emits energy pulses, such as ultrasound pulses, and receives echo signals from those pulses after they are reflected by body tissue (tissue, fat, bone, vessel, plaque, etc., or other object). If desired, the imaging transducer may emit energy pulses while simultaneously rotating about a central axis or translate longitudinally along the central axis. The imaging console 20 receives and processes the echo signals from the imaging transducer assembly 1 to form a cross-sectional image (e.g., an intravascular ultrasound ("IVUS") image) on a display screen (not shown).

In addition to acquiring the image, as disclosed in WO 00/19907 it is often desirable for the operator to be able to drive the work flow of the imaging system as well. For example, the operator may want to input patient records, lab records, and patient registration information prior to obtaining imaging information. Accordingly, an improved imaging system is desirable.

### SUMMARY OF THE INVENTION

The present invention is generally directed to medical imaging systems, and more particularly to a control panel for a medical imaging system. In one embodiment, a medical imaging system includes an imaging display device, an imaging/acquisition processor communicatively coupled to the imaging display device, and an imaging catheter communicatively coupled to the imaging display device and imaging/acquisition processor. The imaging system further includes a control panel communicatively coupled to the imaging display device and imaging/acquisition processor, wherein the control panel includes a first control mechanism for controlling workflow and a second control mechanism for controlling imaging. The first control mechanism is a touch screen display device configured to display a first interface and a second interface according to present claim 1.

In one embodiment of the invention the first control mechanism is a touch screen display device and the second control mechanism is a touch pad and select button.

Other systems, methods, features and advantages of the invention will be or will become apparent to one with skill in the art upon examination of the following figures and detailed description. It is intended that all such additional systems, methods; features and advantages be included within this description, be within the scope of the invention, and be protected by the accompanying claims.

### BRIEF DESCRIPTION OF THE DRAWINGS

In order to better appreciate how the above-recited and other advantages and objects of the inventions are obtained, a more particular description of the embodiments briefly described above will be rendered by reference to specific embodiments thereof, which are illustrated in the accompanying drawings. It should be noted that the components in the figures are not necessarily to scale, emphasis instead being placed upon illustrating the principles of the invention. Moreover, in the figures, like reference numerals designate corresponding parts throughout the different views. However, like parts do not always have like reference numerals. Moreover, all illustrations are intended to convey concepts, where relative sizes, shapes and other detailed attributes may be illustrated schematically rather than literally or precisely.
Fig. 1 is a system diagram of a medical imaging system known in the art.
Fig. 2 is an illustration of a medical imaging system in accordance with a preferred embodiment of the present invention.
Fig. 3 is an illustration of another medical imaging system in accordance with a preferred embodiment of the present invention.
Fig. 4a is a perspective view of a control panel in accordance with a preferred embodiment of the present invention.
Fig. 4b is a rear view of a control panel in accordance with a preferred embodiment of the present invention.
Fig. 5 is a circuit diagram of a control panel in accordance with a preferred embodiment of the present invention.
Fig. 6a is an example user interface in accordance with a preferred embodiment of the present invention.
Fig. 6b is another example user interface in accordance with a preferred embodiment of the present invention.

### DETAILED DESCRIPTION OF THE PREFERRED EMBODIMENTS

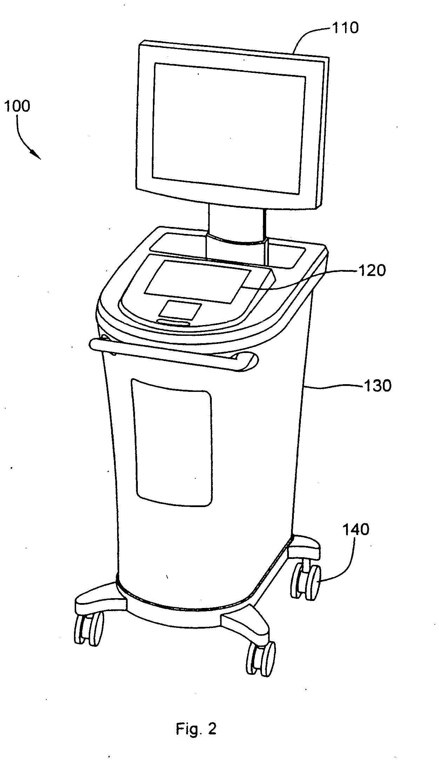

Turning to Fig. 2, an imaging system 100 having a control panel 120 in accordance with an embodiment of the invention is shown. The imaging system 100 includes a display monitor 110, for displaying the images, coupled to an imaging console (not shown) encased within a mobile platform 130 supported by a set of wheels 140. This imaging system 100 enables an operator to quickly optimize the position of the system 100 around the patient or efficiently transfer the imaging system 100 to another room.

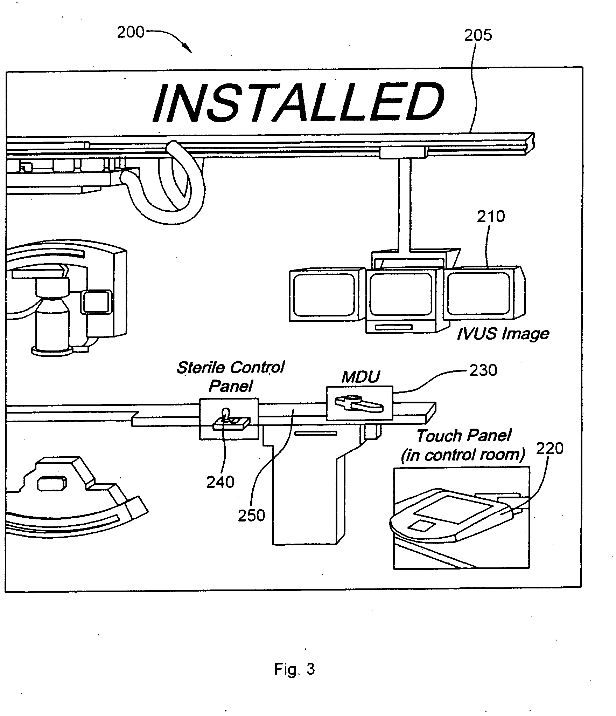

An alternative imaging system 200 is shown in Fig. 3 having the processor (not shown) and the display monitor 210, for displaying medical images, installed on an articulating arm 205 in a catheter laboratory. The patient's table 250 includes a sterile control panel 240 for controlling the imaging device, and a motor drive unit ("MDU") 230, which couples the imaging catheter (not shown) to the imaging/aquisition processor. A more detailed description of the MDU is provided in U.S. Patent 6,261,246, filed on September 28, 1998. The imaging system 200 further includes a control panel 220, typically located in a control room outside of the catheter laboratory.

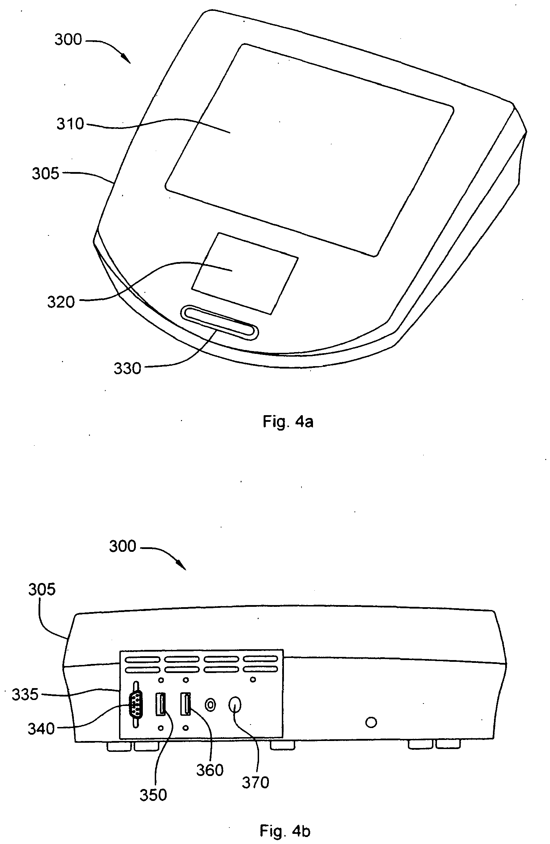

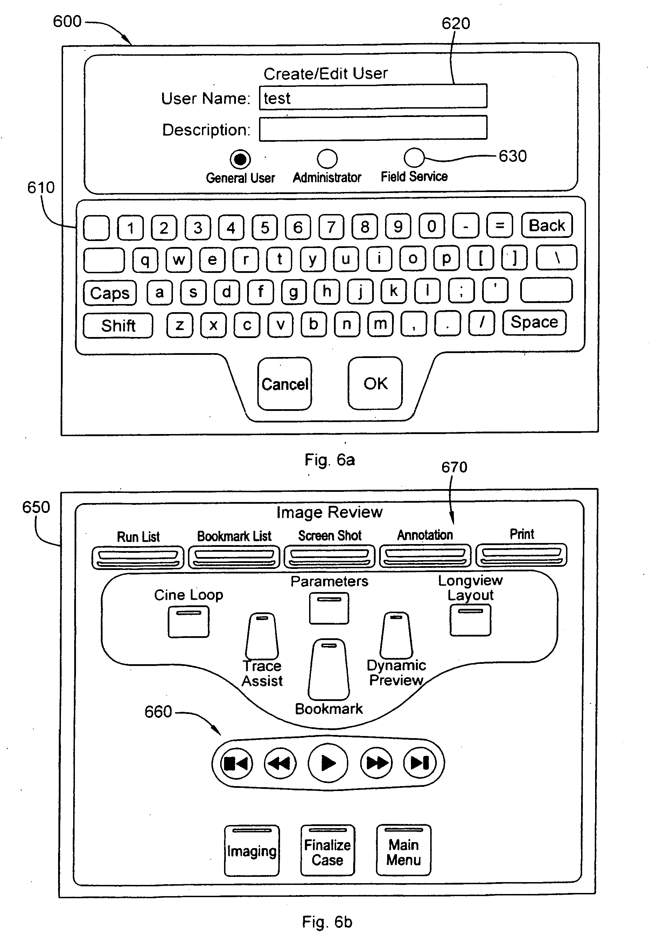

Turning to Fig. 4a, a control panel 300 in accordance with a preferred embodiment is shown. The control panel 300 includes a touch screen display 310, a touch pad 320, and a button 330, all located on the top of a casing 305. The touch screen display 310 provides workflow and workflow information during the operation of the imaging system, e.g., 100 or 200. Workflow and workflow information can include patient records, lab record management, data retrieval, patient check-in and registration, and image processing and playback functions. Turning to Fig. 6a, an example programmable graphical interface 600 provided in the touch screen display 310 that enables workflow is shown. The interface 600 includes text boxes 620, radio buttons 630, and a graphical keyboard 610 that allow a user to input data into the imaging system, such as user information. What is shown is an interface 600 that enables a user to establish a user name and user type, e.g., general user, administrator, or field service. Such data is preferably stored in a host computer or a central computer system on a network accessible by the control panel 300, as will be described below.

Turning to Fig. 6b, another programmable interface 650 is shown, which enables a user to control imaging operations 670, such as establishing bookmarks, changing view perspective, taking screen shots, printing, etc.. In addition, the interface 650 can provide imaging playback functions.

In addition to the touch screen display 310, a touch pad 320 and button 330 are also provided, which provide similar functionality to that of a mouse, enabling an operator to control a pointer on the monitor displaying the medical image, e.g., 110 or 210, and to select regions of the medical image being displayed.

Turning to Fig. 4b, a rear view of the control panel 300 is shown having an input/output ("I/O") panel 335. The I/O panel 335 provides a connection 340 between the control panel 300 and an imaging display monitor, such as a video graphics array ("VGA") monitor, e.g., 110 or 210. The I/O panel 335 further provides a connection 350 between the control panel 300 and an imaging processor, which is preferably a universal serial bus ("USB") connection. The imaging processor can be in the form of a host computer (not shown) or a computer system on a network (not shown), which can provide not only image processing functionality but also workflow information, such as a database of patient records and other medical images. A second USB connection 360 is provided for additional peripheral devices, such as a mouse, which can be used in lieu of the touch pad 320 and button 330. A power supply connection 370 is also included, which is preferably a 12 VDC connection.

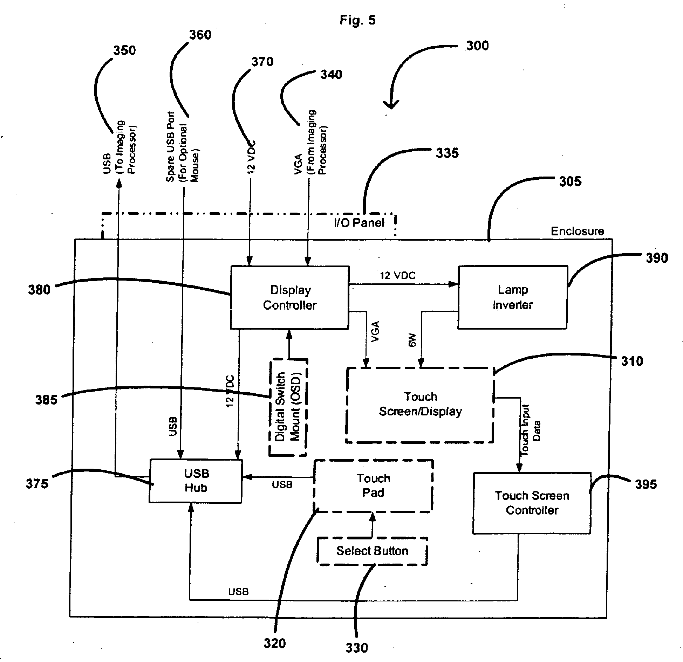

Turning to Fig. 5, an illustration of the circuitry within the control panel 300 is shown. The panel 300 includes a display controller 380 coupled to a lamp inverter 390 and a USB hub 375, which is coupled to the touch pad 320 and the button 330. The display controller 380 is further coupled to the touch screen display 310. The lamp inverter 390 is also coupled to the touch screen display 310, which is also coupled to a touch screen controller 395, which is coupled to the USB hub 375. Each of these components can be industry standard components.

The display controller 380 is further coupled to the 12VDC power supply 370, which provides power to the display controller 380, the lamp inverter 390, and the USB hub 375. The display controller 380 processes video signals, e.g., VGA signals, from the imaging processor to the imaging display monitor, e.g., 110 or 210. The lamp inverter 390 processes the power input and provides fluorescent power signals to backlights for the touch screen display 310. The USB hub 375 provides a single interface for sharing USB signals between the touch screen controller 395 and the touch pad 320 and button 330. The control panel 300 further includes an on-screen display ("OSD") device 385 coupled to the display controller 380 for controlling the display settings, such as brightness and contrast, for the imaging monitor, e.g., 110 or 210, and/or the touch screen display 310.

The touch screen controller 395 interprets touch commands from the user via the touch screen display 310 and the touch pad 320 and button 330 and sends out corresponding USB signals to the imaging processor. The control panel 300 described provides a user multiple points of system control for the user's convenience. Further, the graphical interface, e.g., 600 and 650, supports an intuitive, structured, and programmable workflow, which allows the operator to manage patient data in conjunction with operating the imaging device, as one of ordinary skill in the art would appreciate. Further, the control panel 300 enables exporting to patient data for offline viewing and analysis.

In the foregoing specification, the invention has been described with reference to specific embodiments thereof. It will, however, be evident that various modifications and changes may be made thereto. For example, the reader is to understand that the specific ordering and combination of process actions described herein is merely illustrative, and the invention can be performed using different or additional process actions, or a different combination or ordering of process actions. For example, this invention is particularly suited for applications involving medical imaging devices, but can be used on any design involving imaging devices in general. As a further example, each feature of one embodiment can be mixed and matched with other features shown in other embodiments. Additionally and obviously, features may be added or subtracted as desired. Accordingly, the invention is not to be restricted except in light of the attached claims and their equivalents.

## Claims

1. A medical imaging system (100; 200) comprising:
an imaging display device (110; 210);
an imaging/acquisition processor communicatively coupled to the imaging display device;
an imaging catheter communicatively coupled to the imaging display device and imaging/acquisition processor; and
a control panel (120; 220; 300) communicatively coupled to the imaging display device (110; 210) and imaging/acquisition processor, wherein the control panel includes a first control device for controlling workflow and a second control device **characterised in that** the first control device is a touch screen display device (310) configured and arranged to display a first interface (600) and a second interface (650), and **in that** the first interface (600) comprises a keyboard (610) to permit a user to input data into the imaging system and the second interface comprises controls to enable a user to control imaging operations (670) and provide imaging playback functions.

2. The medical imaging system of claim 1, wherein the second control device is a touch pad (220; 320) that is separate from the touch screen display device (310).

3. The medical imaging system of claim 1, further comprising a motor drive unit coupled to the imaging catheter.

4. The medical imaging system of claim 1, wherein the first and second control devices are coupled to a USB hub (375) within the control panel (120; 220; 300).

5. The medical imaging system of claim 1, wherein the control panel (120; 220; 300) further includes a lamp inverter (390) coupled to the touch screen display device (310).

6. The medical imaging system of claim 1, wherein the control panel (120; 220; 300) further includes an on screen display device configured to enable a user to control display settings for the imaging display device (110; 210) and the touch screen display device (310).

7. The medical imaging system of claim 1, wherein the control panel (120; 220; 300) further includes a display controller (380) coupled to the imaging display device and the touch screen display device (310).

8. The medical imaging system of claim 1, wherein the imaging/acquisition processor is a host computer.

9. The medical imaging system of claim 8, wherein the host computer includes a database of patient records to be displayed on the touch screen display device (310).

10. The medical imaging system of claim 1, wherein the medical imaging system is portable.

11. The medical imaging system of claim 1, wherein the medical imaging system is integrated into a catheter laboratory.

12. The medical imaging system of claim 1, further comprising a motor drive unit coupled to the imaging catheter.

## Patentansprüche

1. Medizinisches Abbildungssystem (100; 200), welches aufweist:
eine Abbildungsanzeigevorrichtung (110; 210);
einen Abbildungs-/Erfassungsprozessor, der kommunikativ mit der Abbildungsanzeigevorrichtung gekoppelt ist;
einen Abbildungskatheter, der kommunikativ mit der Abbildungsanzeigevorrichtung und dem Abbildungs-/Erfassungsprozessor gekoppelt ist; und
eine Steuertafel (120; 220; 300), die kommunikativ mit der Abbildungsanzeigevorrichtung (110; 210) und dem Abbildungs-/Erfassungsprozessor gekoppelt ist, wobei die Steuertafel eine erste Steuervorrichtung zum Steuern des Arbeitsablaufs und eine zweite Steuervorrichtung enthält, **dadurch gekennzeichnet, dass** die erste Steuervorrichtung eine Berührungsbildschirm-Anzeigevorrichtung (310) ist, die ausgebildet und angeordnet ist zum Darstellen einer ersten Schnittstelle (600) und einer zweiten Schnittstelle (650), und dass die erste Schnittstelle (600) eine Tastatur (610) aufweist, um einem Benutzer zu ermöglichen, Daten in das Abbildungssystem einzugeben, und die zweite Schnittstelle Steuerungen aufweist, um einem Benutzer zu ermöglichen, Abbildungsvorgänge (670) zu steuern und Abbildungswiedergabefunktionen vorzusehen.

2. Medizinisches Abbildungssystem nach Anspruch 1, bei dem die zweite Steuervorrichtung ein Berührungskissen (220; 320) ist, das von der Berührungsbildschirm-Anzeigevorrichtung (310) getrennt ist.

3. Medizinisches Abbildungssystem nach Anspruch 1, weiterhin aufweisend eine Motorantriebseinheit, die mit dem Abbildungskatheter gekoppelt ist.

4. Medizinisches Abbildungssystem nach Anspruch 1, bei dem die erste und die zweite Steuervorrichtung mit einem USB-Knoten (375) innerhalb der Steuertafel (120; 220; 300) gekoppelt sind.

5. Medizinisches Abbildungssystem nach Anspruch 1, bei dem die Steuertafel (120; 220; 300) weiterhin einen Lampeninverter (390), der mit der Berührungsbildschirm-Anzeigevorrichtung (310) gekoppelt ist, enthält.

6. Medizinisches Abbildungssystem nach Anspruch 1, bei dem die Steuertafel (120; 220; 300) weiterhin eine Onscreen-Anzeigevorrichtung enthält, die ausgebildet ist, einem Benutzer die Steuerung von Anzeigeeinstellungen für die Abbildungsanzeigevorrichtung (110; 210) und die Berührungsbildschirm-Anzeigevorrichtung (310) zu ermöglichen.

7. Medizinisches Abbildungssystem nach Anspruch 1, bei dem die Steuertafel (120; 220; 300) weiterhin eine Anzeigesteuervorrichtung (380) enthält, die mit der Abbildungsanzeigevorrichtung und der Berührungsbildschirm-Anzeigevorrichtung (310) gekoppelt ist.

8. Medizinisches Abbildungssystem nach Anspruch 1, bei dem der Abbildungs-/Erfassungsprozessor ein Hostcomputer ist.

9. Medizinisches Abbildungssystem nach Anspruch 8, bei dem der Hostcomputer eine Datenbank für Patientenaufzeichnungen, die auf der Berührungsbildschirm-Anzeigevorrichtung (310) anzuzeigen sind, enthält.

10. Medizinisches Abbildungssystem nach Anspruch 1, bei dem das medizinische Abbildungssystem tragbar ist.

11. Medizinisches Abbildungssystem nach Anspruch 1, bei dem das medizinische Abbildungssystem in ein Katheterlabor integriert ist.

12. Medizinisches Abbildungssystem nach Anspruch 1, weiterhin aufweisend eine Motorantriebseinheit, die mit dem Abbildungskatheter gekoppelt ist.

## Revendications

1. Système d'imagerie médicale (100 ; 200) comprenant :
un dispositif d'affichage d'imagerie (110 ; 210) ;
un processeur d'imagerie/d'acquisition couplé en communication au dispositif d'affichage d'imagerie ;
un cathéter d'imagerie couplé en communication au dispositif d'affichage d'imagerie et au processeur d'imagerie/d'acquisition ; et
un panneau de commande (120 ; 220 ; 300) couplé en communication au dispositif d'affichage d'imagerie (110 ; 210) et au processeur d'imagerie/d'acquisition, dans lequel le panneau de commande inclut un premier dispositif de commande pour commander un flux de travail et un second dispositif de commande, **caractérisé en ce que** le premier dispositif de commande est un dispositif d'affichage à écran tactile (310) configuré et agencé pour afficher une première interface (600) et une seconde interface (650) et **en ce que** la première interface (600) comprend un clavier (610) pour permettre à un utilisateur d'entrer des données dans le système d'imagerie et la seconde interface comprend des commandes pour permettre à un utilisateur de commander des opérations d'imagerie (670) et pour assurer des fonctions de relecture d'imagerie.

2. Système d'imagerie médicale selon la revendication 1, dans lequel le second dispositif de commande est un pavé tactile (220 ; 320) qui est séparé du dispositif d'affichage à écran tactile (310).

3. Système d'imagerie médicale selon la revendication 1, comprenant en outre une unité de commande de moteur couplée au cathéter d'imagerie.

4. Système d'imagerie médicale selon la revendication 1, dans lequel les premier et second dispositifs de commande sont couplés à un hub USB (375) dans le panneau de commande (120 ; 220 ; 300).

5. Système d'imagerie médicale selon la revendication 1, dans lequel le panneau de commande (120 ; 220 : 300) inclut en outre un inverseur à lampe (390) couplé au dispositif d'affichage à écran tactile (310).

6. Système d'imagerie médicale selon la revendication 1, dans lequel le panneau de commande (120 ; 220 ; 300) inclut en outre un dispositif d'affichage sur écran configuré pour permettre à un utilisateur de commander des réglages d'affichage pour le dispositif d'affichage d'imagerie (110 ; 210) et le dispositif d'affichage à écran tactile (310).

7. Système d'imagerie médicale selon la revendication 1, dans lequel le panneau de commande (120 ; 220 ; 300) inclut en outre un contrôleur d'affichage (380) couplé au dispositif d'affichage d'imagerie et au dispositif d'affichage sur écran (310).

8. Système d'imagerie médicale selon la revendication 1, dans lequel le processeur d'imagerie/d'acquisition est un ordinateur hôte.

9. Système d'imagerie médicale selon la revendication 8, dans lequel l'ordinateur hôte inclut une base de données d'enregistrements de patient à afficher sur le dispositif d'affichage à écran tactile (310).

10. Système d'imagerie médicale selon la revendication 1, dans lequel le système d'imagerie médicale est portable.

11. Système d'imagerie médicale selon la revendication 1, dans lequel le système d'imagerie médicale est intégré dans un laboratoire de cathéter.

12. Système d'imagerie médicale selon la revendication 1, comprenant en outre une unité de commande de moteur couplée au cathéter d'imagerie.
